**Europäisches Patentamt**

**European Patent Office**

**Office européen des brevets**

(19)

(11) Veröffentlichungsnummer: **0 005 713**
**B1**

(12) ## EUROPÄISCHE PATENTSCHRIFT

(45) Veröffentlichungstag der Patentschrift:
**23.01.85**

(51) Int. Cl.⁴: **A 61 N 1/42**

(21) Anmeldenummer: **79101056.4**

(22) Anmeldetag: **03.05.79**

(54) **Magnetfeldbehandlungsgerät.**

(30) Priorität: **06.06.78 DE 2824698**

(43) Veröffentlichungstag der Anmeldung:
**12.12.79 Patentblatt 79/25**

(45) Bekanntmachung des Hinweises auf die Patenterteilung:
**23.01.85 Patentblatt 85/4**

(84) Benannte Vertragsstaaten:
**BE CH DE GB IT LU NL SE**

(56) Entgegenhaltungen:
**BE - A - 658 060**
**DE - A - 1 464 894**
**DE - A - 2 116 869**
**DE - A - 2 603 934**
**DE - A - 2 707 574**
**DE - A - 2 716 672**
**FR - A - 717 001**
**FR - A - 1 331 996**
**FR - A - 2 178 242**
**FR - A - 2 370 484**
**GB - A - 735 290**
**GB - A - 1 164 119**

(73) Patentinhaber: **Rasche, Erich, Am Jinglingsberg 8,
D-7632 Friesenheim 3 (DE)**
Patentinhaber: **Ludwig, Wolfgang, Dr.rer.nat.
Diplomphysiker, Quellgasse 7, D-7400 Tübingen-1 (DE)**

(72) Erfinder: **Rasche, Erich, Am Jinglingsberg 8,
D-7632 Friesenheim 3 (DE)**
Erfinder: **Ludwig, Wolfgang, Dr.rer.nat. Diplomphysiker,
Quellgasse 7, D-7400 Tübingen-1 (DE)**

BUNDESDRUCKEREI BERLIN

**Beschreibung**

Die Erfindung betrifft ein elektronisches Gerät zur Behandlung von Patienten, wobei magnetische Wechselfelder verwendet werden.

Je nach beabsichtigter Wirkung werden entweder natürliche magnetische Wechselfelder (wie sie z. B. der menschliche Organismus erzeugt und wie sie beispielsweise als Magnetoencephalogramm abgreifbar sind) mittels eines oder mehrerer magnetischer Dipole aufgefangen und gegebenenfalls zeitversetzt und/oder verstärkt einem zweiten magnetischen Dipol oder mehreren weiteren magnetischen Dipolen zugeführt, oder es werden künstlich erzeugte magnetische Wechselfelder verwendet.

Das Gerät arbeitet im Frequenzbereich von 0 bis 1 Gigaherzt, vorzugsweise bis 1 Megahertz. Die verwendete magnetische Induktion liegt im Bereich von Nanotesla bis etwa einem Tesla. Die beabsichtigte Wirkung ist ein Einfluß auf das biologische Geschehen im Organismus des Patienten.

Vorläufer des erfindungsgemäßen Gerätes sind ähnliche Anordnungen, die mit elektrischen Wechselfeldern oder Wechselströmen arbeiten und z. B. als Reizstromgeräte bekannt sind. Außerdem sind Magnetfeldtherapiegeräte mit einem oder zwei magnetischen Dipolen bekannt, wobei diese Dipole in Serie oder parallel geschaltet sind, also kein nennenswerter Phasen- und/oder Amplitudenunterschied zwischen beiden Dipolsignalen besteht.

Die Wirkung der bekannten Geräte ist auf bestimmte Indikationen beschränkt und die Erfolgsquote liegt unter 85%. Ein Nachteil bisheriger Anordnungen ist außerdem, daß die therapeutisch wirksame Frequenz durch Versuche erprobt werden muß.

Die vorliegende Anmeldung stellt sich die Aufgabe, das Wirkungsspektrum und die Erfolgsquote der bisherigen Geräte zu erhöhen, neue Anwendungsbereiche zu erschließen und eine automatische Frequenzeinstellung zu ermöglichen. Die hierzu vorgeschlagene Lösung dieser Aufgabe, wie in der Kennzeichnung des Patentanspruchs 1 definiert, ist erprobt und hat sich bereits bei Testversuchen bewährt. Insbesondere soll das erfindungsgemäße Gerät folgende Möglichkeiten aufweisen:

1. Auffangen magnetischer Wechselfelder mittels eines magnetischen Dipols, verstärken der vom Dipol abgegebenen Wechselspannung, einstellbar im Bereich von 0 bis mindestens 90 db, Zeitverzögerungen des Signals, einstellbar zwischen 0 und 1/f Sekunden mit f = gewählte unterste Grenzfrequenz, sowie Umsetzen des so erhaltenen Signals in ein magnetisches Wechselfeld mittels weiterer magnetischer Dipole. Bei der medizinischen Anwendung wird dabei die Zeitverzögerung so gewählt, daß Ein- und Ausgangssignal um jene Zeit gegeneinander versetzt sind, die sich aus der Nervenleitgeschwindigkeit im Organismus zwischen Angriffstelle und Therapiestelle (z. B. Abgriff am Kopf, Therapie am Brustkorb) ergibt.

2. Erzeugen zweier im Fourierspektrum exakt oder nahezu gleicher magnetischer Wechselfelder mittels eines ersten magnetischen Dipols und eines zweiten Dipols oder einer Gruppe zweiter magnetischer Dipole, wobei zwischen beiden Feldern ein einstellbarer Amplitudenunterschied und/oder eine einstellbare Zeitverzögerung bestehen, die im Falle Medizinischer Anwendung wie bei 1. mit Nervenleitgeschwindigkeiten korrelierbar sind (z. B. erstes Feld in Kopfnähe, zeitverzögertes Feld in Brustnähe).

3. Automatische elektronische Steuereinrichtungen zur Einstellung von Frequenz- und Zeitverzögerung, wobei als Detektor im Falle medizinischer Anwendung Hauttemperaturfühler oder Hautwiderstandssonden verwendet werden können (z. B. Temperaturfühler in der Armbeuge als HEADschen Zone des Kreislaufregulationszentrums oder im Mund. Bei einem Anstieg der Temperatur um ca. 0,5 Grad Celsius wird die langsam von ca. 1 Hertz hochlaufende Frequenz des Wechselstromgenerators automatisch festgehalten, erfahrungsgemäß zwischen 5 und 12 Hertz). Freqenz- und Zeitverzögerungssteuerung können nacheinander erfolgen, beispielsweise durch automatisches Umschalten.

4. Feldlinienkonzentration an gewünschten Orten durch Situierung von hochpermeablem Material (z. B. MU-Metall) im wirksamen Feldbereich der magnetischen Dipole.

Vorteile der beschriebenen Erfindung sind:

Breiterer Indikationsbereich als bisher möglich, Beispielsweise ist durch die unter 1., 2. und 4. genannten Prinzipien eine spezifische Behandlung eines bestimmten Organs im menschlichen Organismus möglich.

Höhere Erfolgsquote, die erfahrungsgemäß in vielen Anwendungsbereichen 98% erreicht.

Einfachste Bedienung durch Steuerautomatik für Frequenz- und Zeitverzögerungswahl.

Vermeidung ungünstiger Einstellungen mit negativen Wirkungen durch das unter 3. genannte Prinzip, das automatisch die günstigste Einstellung des Gerätes findet.

Die Magnetfeldtherapie mit den aufgezeigten Prinzipien hat folgende Konsequenzen:

Impulsförmige Feldänderungen (z. B. nadelförmige »Spices« hoher Flankensteilheit und geringer Impulsbreite) induzieren in organischem Gewebe Wirbelströme, wobei eine positive Wirkung von Strömen in der Größenordnung von Mikroampére und mit einer Folgefrequenz im Frequenzbereich eines Elektro- bzw. Magnetoencephalogramms bekannt ist (vgl. in Betracht gezogene Druckschriften). Der Vorteil gegenüber elektrischen Feldern, nämlich die hohe Eindringtiefe magnetischer Felder bis ins Knochenmark, ist ebenfalls bekannt.

Die im Organismus induzierten Wirbelströme rufen im Nervensystem Miniaturpotentiale hervor, die sich an Synapsen zu Aktionspotentialen aufsummieren. Zufolge der endlichen Nervenleitungsgeschwindigkeit bestehen Zeitverschiebungen zwischen äußeren künstlichen Feldimpulsen und den örtlichen Nervenimpulsen im Körper. Ebenso bestehen in einzelnen Körperpartien zwischen Teilen des Nervensystems Impulszeitunterschiede.

Um einen bestimmten Körperteil gezielt zu behandeln, sollten daher diese Zeitdifferenzen — die für verschiedene Nervenbahnen verschieden groß sind, so daß eine bestimmte Nervenbahn oder mehrere Nervenbahnen speziell angesprochen werden kann bzw. können — berücksichtigt werden, wie es oben unter 2. beschrieben wurde. Dies gilt sowohl für die Behandlung mit (verstärkten) körpereigenen Impulsen als auch mit an zwei verschiedenen Orten des Organismus induzierten fremden Impulsen.

Je nach behandeltem Organ (entsprechend der räumlichen Anordnung der magnetischen Dipole und der Zeitverzögerung zwischen beiden) erhöht sich die Hauttemperatur bzw. sinkt der Hautübergangswiderstand an bestimmten HEADschen Zonen. Dies kann zur Ermittlung der passenden Impulsfolgefrequenz herangezogen werden, wie oben unter 3. beschrieben. Nach (automatisch gesteuerter) Optimierung der Impulsfolgefrequenz können die Zeitverzögerungen, die aufgrund von Erfahrungswerten vorjustiert wurden, ebenfalls automatisch nacheinander optimiert werden.

Um an bestimmten Körperregionen in Hautnähe die Feldstärke örtlich zu vergrößern, können dort Plättchen aus hochpermeablem ferromagnetischem Material (z. B. Mu-Metall mit Heftpflaster befestigt) angeheftet werden.

Die Vorteile dieser Maßnahme seien hier nochmals speziell für den medizinischen Sektor zusammengestellt:

- gezielte Behandlungsmöglichkeit bestimmter Körperregionen bzw. Organe
- optimale Behaldung mit der physiologisch wirksamsten Impulsfolgefrequenz und damit Wegfall von Nebenwirkungen, wie sie bei physiologisch ungünstigen Impulsfolgefrequenzen auftreten können.
- einfachste Bedienung durch programmierbaren Ablauf der Gerätesteuerung zur automatischen Einstellung von Impulsfolgefrequenz und Zeitverzögerung
- Möglichkeit der unmittelbar aufeinanderfolgenden Behandlung mit körpereigenen und fremden Impulsen und damit erfahrungsgemäß Erhöung der Erfolgsquote
- ein weiterer wesentlicher Vorteil, der sich in praxi bewährt hat, ist die Möglichkeit, das verwendete ferromagnetische Material (als Kern der magnetischen Dipole oder als Schirm um die magnetischen Dipole nach außen (vom Patienten abgekehrt) sowie als Plättchen zur Feldlinienkonzentration) nach bestimmten Verfahren vorzubehandeln, was jedoch nicht Gegenstand der vorliegenden Anmeldung ist.

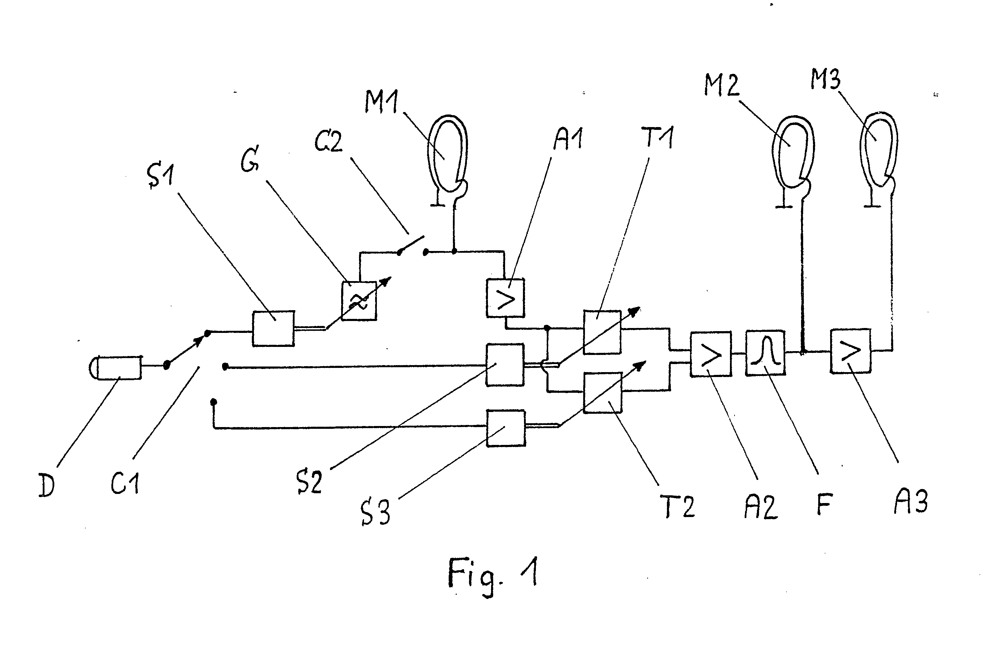

Fig. 1 zeigt das Prinzipschaltbild des Gerätes, wobei nur ein magnetischer Dipol an der Aufnahmeseite (links in Fig. 1) gezeichnet wurde.

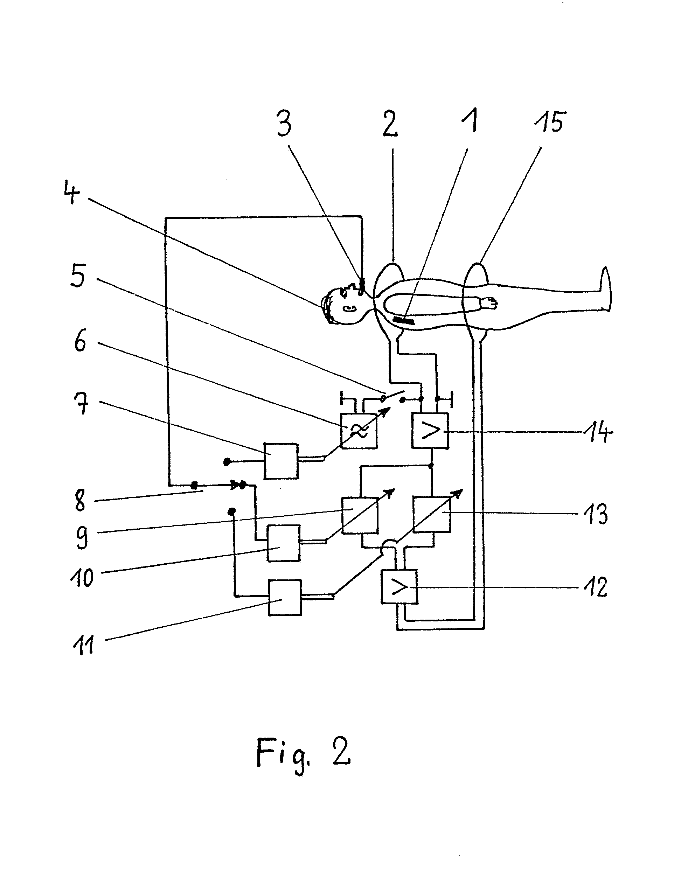

Anhand von einem Beispiel, Fig. 2, sollen das Gerät und seine Anwendung in der Medizin beschrieben werden. Beispielsweise soll die Leber des Patienten behandelt werden. Die HEADsche Zone der Leber befindet sich in Nähe der Schulterblätter, weshalb dort ein (vorbehandeltes) hochpermeables ferromagnetisches Plättchen (1) zur örtlichen Feldlinienkonzentration angeheftet wird.

Abgegriffen werden die körpereigenen Signale im gleichen Bereich mit dem ersten magnetischen Dipol (2). Die Messung der Temperatur geschieht mit dem Detektor (3) im Mund des Patienten (4). Der Wahlschalter (5) ist zunächst auf »Eigenbehandlung« gestellt, d. h. der erste magnetische Dipol (2) wird als Empfangssystem benutzt und speist den Verstärker (14), der ein oder mehrere elektronische Frequenzfilter enthalten kann, die gewünschte Frequenzbereiche ausfiltern oder Störungen unterdrücken. Der Wechselstromgenerator (6) ist dabei ausgeschaltet, so daß auch das Steuersystem (7) für dessen Frequenz vorerst ohne Funktion bleibt. Mit dem Umschalter (8) ist der Detektor (3) auf den Eingang des Steuersystems (10) für die erste Zeitverzögerung geschaltet. Beide Zeitverzögerungsstrecken (9) und (13) werden aufgrund von Erfahrungswerten vorjustiert und zwar absichtlich auf zu kleine Zeiten eingestellt, um den Steuersystemen (10) und (11) Gelegenheit zu geben, in den entsprechenden Schalterstellungen des Umschalters (8) nacheinander die Zeitverzögerungen zu optimieren. Dabei werden verschiedene Nervenbahnen mit unterschiedlichen Nervenleitgeschwindigkeiten berücksichtigt. Es können ohne weiteres noch weitere Zeitverzögerungsstrecken außer (9) und (13) mit entsprechenden weiteren Steuersystemen eingebaut werden, die alle auf einen Summierverstärker (12) geschaltet werden. Der Summierverstärker (12) addiert die unterschiedlich zeitverzögerten Eingangssignale und speist im Ausgang den zweiten magnetischen Dipol (15), der stets als Sendesystem wirkt und im Beispiel den Bezirk der Leber behandelt. Er kann in zwei Orientierungen zum ersten magnetischen Dipol (2) verwendet werden: gleich- und gegenphasig.

Die Verstärkung des Verstärkers (14) wird nach Erfahrungswerten eingestellt. Nach einer Behandlung wie eben beschrieben, die in der Regel zwischen circa einer Minute und circa zwanzig Minuten liegt, kann mit dem Wahlschalter (5) auf Fremdbehandlung umgeschaltet werden.

Dazu ist der Wechselstromgenerator (6) einzuschalten und der Umschalter (8) zunächst auf das Steuersystem (7) zu schalten. Diese Umschaltung kann mittels eines automatisch ablaufenden Programmschalters erfolgen. In dieser neuen Einstellung des Wahlschalters (5) arbeiten beide magnetischen Dipole (2) und (15) als Sendesysteme, jedoch gegebenenfalls mit unterschiedlicher Amplitude und unterschiedlich zeitverzögert. Bei Verwendung zweier Zeitverzögerungsstrecken (9), (13) und eventuell weiterer erhält der magnetische Dipol (15) ein Signalgemisch, das aus dem Signal des Wechselstromgenerators (6) durch unterschiedliche Zeitverzögerung gebildet wird.

Solange der Umschalter (8) auf das Steuersystem (7) geschaltet ist, wird die Sendefrequenz des Wechselstromgenerators (6) zunächst auf eine tiefe Erfahrungsfrequenz, zum Beispiel ein Hertz gestellt und mit dem Steuersystem (7) langsam höher geregelt. Sobald der Detektor (3) einen leichten Temperaturanstieg an den Eingang des Steuersystems (7) meldet, stoppt das Steuersystem (7) das Hochregeln der Frequenz ab und hält die erreichte Frequenz des Wechselstromgenerators (6) fest.

Da solche Temperaturerhöhungen an HEADschen Zonen — im Beispiel im Mund des Patienten (4) — eine längere Nachwirkungszeit haben, kann bei der ersten Behandlung die Optimierung der Einstellung der Zeitverzögerungsstrecken (9), (13) mittels der Steuersysteme (10) und (11) noch nicht genau genug erfolgen. In praxi erhält man nach zwei bis drei Behandlungen so genaue Erfahrungswerte, daß das Gerät zu Folgebehandlungen für den betreffenden Patienten (4) bereits weitgehend vorjustiert werden kann. Ist zum Beispiel die physiologisch günstigste Frequenz des Wechselstromgenerators (6) für den Patienten (4) bereits bekannt, so kann der Umschalter (8) gleich zu Beginn auf das Steuersystem (10) gestellt werden und die erste Zeitverzögerungsstrecke (9) mit null Sekunden Verzögerungszeit beginnend kontinuierlich zu höheren Verzögerungszeiten geregelt werden. Die zweite Verzögerungsstrecke (13) ist hierbei abgeschaltet. Bei optimaler Verzögerungsstrecke (9) steigt die Temperatur in der Mundhöhle des Patienten (4) erneut leicht an, so daß über den Detektor (3) ein Haltesignal im Steuersystem (10) erzeugt werden kann, das die gerade erreichte Verzögerungszeit der ersten Zeitverzögerungsstrecke (9) festhält. Analog wird mit der zweiten Zeitverzögerungsstrecke (13), gesteuert durch das Steuersystem (11) verfahren. Dabei bleibt die erste Zeitverzögerungsstrecke (9) eingeschaltet.

Für die folgenden Behandlungen des Patienten (4) werden die für ihn gefundenen optimalen Einstellungen von Wechselstromgenerator (6) und erster sowie gegebenenfalls zweiter Zeitverzögerungsstrecke (9), (13) — die durch eingebaute Skalen angezeigt und schriftlich festgehalten werden können — von Hand eingestellt. Die Steuersysteme (7), (10), (11) und eventuell weitere für zusätzliche Zeitverzögerungsstrecken bleiben dann eingeschaltet und die Verwendung des Detektors (3) ist nicht notwendig.

Es hat sich gezeigt, daß für jeden Patienten die für ihn gefundenen Einstellungen zeitlich konstant gehalten werden können. Bei der Betätigung des Wahlschalters (5) brauchen die gefundenen optimalen Einstellungen der Zeitverzögerungsstrecken (9), (13) nicht geändert zu werden. Dies ist ein Vorteil der Anordnung.

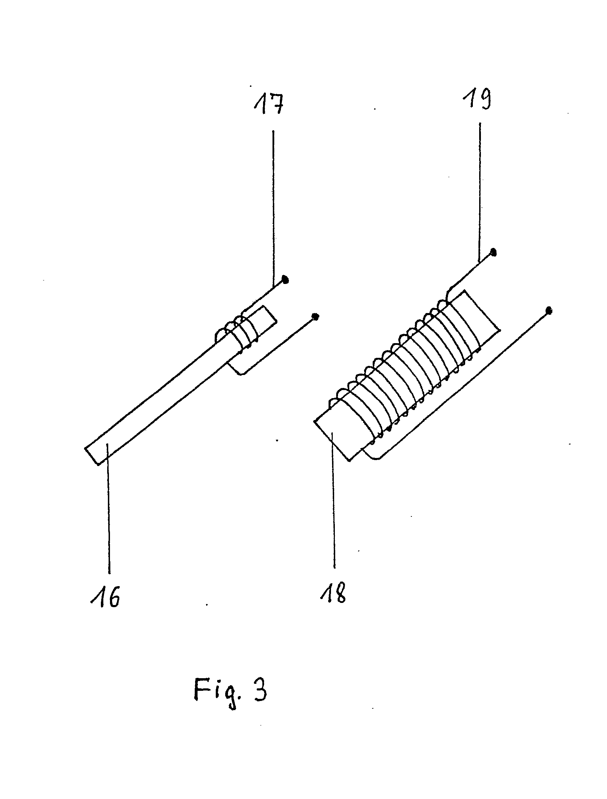

Fig. 3 zeigt weitere Beispiele für Ausführungen der magnetischen Dipole, die in Fig. 2 mit (2) beziehungsweise (15) bezeichnet wurden. Links in Fig. 3 ist ein Ferritstab (16) mit aufgewikkelter Spule (17) gezeigt, der vom Patienten in die Hand genommen wird. Rechts in Fig. 3 ist ein ferromagnetisches Blech (18) mit aufgewickelter Spule (19) gezeigt. Diese Anordnung kann an den gewünschten Orten flach auf die Haut des Patienten gelegt werden.

Für besondere Anwendungen kann der Wechselstromgenerator (6) in Fig. 2 mehrere Signale unterschiedlicher Frequenz gleichzeitig liefern.

Legende zu Fig. 1:

| | | |
|---|---|---|
| D | = | Detektor für Temperatur oder elektrische Leitfähigkeit |
| M1, M2, M3 | = | magnetische Dipole |
| S1, S2, S3 | = | (elektrische) Steuersysteme |
| C1 | = | (automatischer) Umschalter für Frequenz- und Zeitverzögerungs-Justierung |
| C2 | = | Wahlschalter für Fremd- und/oder Eigenbehandlung |
| G | = | Wechselstromgenerator |
| A1 | = | Verstärker |
| A2 | = | Summierverstärker |
| A3 | = | Trennverstärker oder Abschwächer |
| F | = | Frequenzfilter zur Unterdrückung von technischen Störungen oder zur gewünschten Änderung des Frequenzganges der verwendeten Verstärker |
| T1, T2 | = | Zeitverzögerungsstrecken |

Legende zu Fig. 2:

In Klammer sind die Bezeichnungen von Fig. 1 angegeben.

| | | |
|---|---|---|
| 1 | = | ferromagnetisches Plättchen |
| 2 | = | erster magnetischer Dipol (M1) |
| 3 | = | Steuerelement (Detektor D) im Mund des Patienten (4) |
| 4 | = | Patient |
| 5 | = | Schalter (C2) |
| 6 | = | Wechselstromgenerator (G) |
| 7 | = | Steuersystem für Generatorfrequenz (S1) |
| 8 | = | Umschalter (C1) |
| 9 | = | erste Zeitverzögerungsstrecke (T1) |

10 = Steuersystem (S2) für erste Zeitverzögerung
11 = Steuersystem (S3) für zweite Zeitverzögerung
12 = Summierverstärker (A2)
13 = zweite Zeitverzögerungsstrecke (T2)
14 = Verstärker (A1)
15 = zweiter magnetischer Dipol (M2)

Literaturhinweise:

1. R. D. Becker and Murray
The electr. Controllsystems, Band 75 (1970)

2. M. Weigert
Anregung der Knochenbildung durch elektr. Strom Heft zur Unfallheilkunde Nr. 115, Springer-Verlag 1972

**Patentansprüche**

1. Magnetfeldbehandlungsgerät zur Behandlung von Patienten, welches mindestens zwei magnetische Dipole (2), (15) umfaßt, dadurch gekennzeichnet, daß einer der magnetischen Dipole (2) wahlweise als Empfänger oder Sender verwendbar ist, wobei dieser Dipol (2) an einen Verstärker (14) angeschlossen ist, und über einen Schalter (5) ein Wechselstromgenerator (6) eingangsseitig des Verstärkers (14) an ihn angeschlossen werden kann, daß eine Steuereinrichtung (7) vorgesehen ist, welche die Frequenz des Wechselstromgenerators automatisch langsam ansteigen läßt, bis der Frequenzanstieg durch ein Signal eines Fühlelementes (3) am Patient angehalten wird, daß die übrigen magnetischen Dipole (15) nur als Sender Verwendung finden und über eine einstellbare Zeitverzögerungsstrecke und einen Verstärker oder mehrere einstellbare Zeitverzögerungsstrecken (9), (13) und einen Summierverstärker (12) mit dem erstgenannten Verstärker (14) verbunden sind, wobei die Verzögerungszeit der Zeitverzögerungsstrecke bzw. -strecken durch eine Steuereinrichtung bzw. durch Steuereinrichtungen (10, 11) solange vergrößert wird, bis diese Vergrößerung durch ein Signal des genannten Fühlelementes (3) unterbrochen wird.

2. Magnetfeldbehandlungsgerät nach Patentanspruch 1, dadurch gekennzeichnet, daß der Wechselstromgenerator (6) ein einstellbares Frequenzgemisch erzeugt.

3. Magnetfeldbehadlungsgerät nach den Patentansprüchen 1 und 2, dadurch gekennzeichnet, daß die Polung der magnetischen Dipole (2, 15) sowohl gleich- als auch gegenphasig zu einander orientiert werden kann, zum Beispiel mit Umpolschaltern.

4. Magnetfeldbehandlungsgerät nach den Patentansprüchen 1 bis 3, dadurch gekennzeichnet, daß das Fühlelement (3) als Detektor für die elektrische Leitfähigkeit oder die Temperatur an einem oder mehreren Ort(en) des Patienten ausgebildet ist.

5. Magnetfeldbehandlungsgerät nach den Patentansprüchen 1 bis 4, gekennzeichnet durch mindestens ein ferromagnetisches Plättchen (1) zur Konzentration der magnetischen Feldlinien an der zu behandelnden Stelle oder an den zu behandelnden Stellen.

6. Magnetfeldbehandlungsgerät nach den Patentansprüchen 1 bis 5, dadurch gekennzeichnet, daß elektronische Filter zur Änderung des Frequenzganges der verwendeten Verstärker eingebaut sind.

**Claims**

1. A magnetic field treating apparatus for the treatment of patients, including at least two magnetic dipoles (2, 15), comprising: one of the magnetic dipoles (2) being optionally used as reciever or transmitter whereby this dipole (2) is connected with an amplifier (14), and an alternator (6) being connected with the input side of the amplifier (14) by a switch (5); a control device (7) being provided enabling the frequency of the alternator to rise slowly and automatically, until the rise of the frequency is stopped by a signal of a sensor (3) attached to the patient; the remaining magnetic dipoles (15) being used as trasmitters only and being connected with the amplifier (14) either by one adjustable time delay section and an amplifier or by several adjustable time delay sections (9, 13) and a summing amplifier (12) whereby the time delay of the time delay sections is steadly increased by a control devices (10, 11) until this increase is broken by a signal of the above mentioned sensor (3).

2. A magnetic field treating apparatus in accordance with claim 1 wherein the alternator (6) produces an adjustable frequency composition.

3. A magnetic field treating apparatus in accordance with claime 1 and 2 wherein the polarity of the magnetic dipoles (2, 15) can be oriented in phase as well as in phase opposition to one another, e. g. by reversing switches.

4. A magnetic field treatment apparatus in accordance with claim 1 to 3 wherein the sensor (3) is formed as detector of the electric conductivity or the temperature in one or more places of the patient.

5. A magnetic field treatment apparatus in accordance with claim 1 to 4 with at least one ferromagnetic plate (1) to concentrate the mangetic field lines in the place or places to be treated.

6. A magnetic field treating apparatus in accordance with claim 1 to 5 wherein electronic filters are installed to alter the frequency response of the amplifier used.

**Revendications**

1. Appareil de traitement par champ magnétique pour soigner des malades. L-appareil comporte au moins deux dipòles magnetiques (2),

(15). Caractéristiques: L'un des dipôles peut servir facultativement de récepteur ou bien d'émetteur, relie alors à un amplificateur (14). Par un interrupteur (5), un alternateur (6) peut ètre raccorde à ce dinòle à l'entree de l'alternateur (14). Un dispositif de reglage est prevu, qui, automatiquement, fait monter peu à peu la frequence de l'alternateur, jusqu' à ce que la montee de la frequence soit arrètee par le signal d'un élément sensitif mis sur le patient. Les autres dipòles magnetiques (15) ne servent aue d'emetteurs et sont relies à l'amplificateur ci-dessus nomme (14) par un element regloble de retandement et un amplificateur ou bien par plusieurs elements reglables de retardement (9), (13) et un amplificateur multiple (12). Le temps de retandement de l'element reglable (des élements réglables) est augmente par un ou plusieurs dispositifs de réglage (10, 11) jusqu'à ce que cette augmentation soit interromnue par un signal de l'élément sesitif (3).

2. Appareil de traitement par champ magnétique selon la demonde de brevet N° 1 caractéristique: L'alternateur (6) produit un mélange de fréquences réglable.

3. Appareil de traitement par champ magnétique selon les demandes de brevet N° 1 et 2 caractéristique: les dipôles magnétiques peuvent être orientés en phase ou en opposition de phase, grâce à des inverseurs par exemple.

4. Appareil de traitement par champ magnétique selon les demandes de brevet N° 1—3 caractéristique: L'élément sensitif (3) sert de détecteur de la conductibilité électrique ou de la température d'un ou plusieurs points du corns du patient.

5. Appareil de traitement par champ magnétique selon les demandes de brevet 1—4, caractérisé par ou moins une plaquette ferro-magnétique qui sert à concentrer les lignes du champ magnétique sur un ou plusieurs points du corps à traiter.

6. Appareil de traitement par champ magnétique selon les demandes de brevet 1—5, caractérisé par des filtres électroniques incorporés qui servent à transformer les fréquences de l'amplificateur utilisé.

Fig. 1

0 005 713

Fig. 2

Fig. 3